# EUROPEAN PATENT APPLICATION

(11) **EP 2 481 397 A1**
(43) Date of publication of application: **01.08.2012**
(21) Application number: 11000678.0
(22) Date of filing: 27.01.2011
(51) Int. Cl.: A61K 9/20, A61K 31/519

(54) **Pharmaceutical compositions comprising tasocitinib**

(71) Applicant: Ratiopharm GmbH, 89070 Ulm (DE)
(72) Inventor: Stefan, Ralph, 88370 Ebenweiler (DE); Meergans, Domonique, 81477 München (DE)
(74) Representative: Aechter, Bernd

(57) **Abstract**

The invention relates to pharmaceutical compositions, preferably tablets, comprising tasocitinib, suitable for immediate release, and processes of preparing such compositions.

## Description

### Background

The present invention is related to pharmaceutical compositions, preferably tablets, comprising 3-{(3R,4R)-4-methyl-3-[methyl-(7H-pyrrolo[2,3-d]pyrimidin-4-yl)-amino]-piperidin-1-yl}3-oxo-propionitrile, suitable for immediate release, and processes of preparing such compositions.

3-{(3R,4R)-4-methyl-3-[methyl-(7H-pyrrolo[2,3-d]pyrimidin-4-yl-amino]-piperidin-1-yl}3-oxo-propionitrile has the chemical formula C₁₆H₂₀N₆O and allegedly is useful as inhibitor of protein kinases, such as the enzyme Janus Kinase 3 (hereinafter also referred to as "JAK3"), and as such it has been asserted that it is useful in therapy as immunosuppressive agents for organ transplants, xenotransplantation, lupus, multiple sclerosis, rheumatoid arthritis, psoriasis, Type I diabetes and complications resulting from diabetes, cancer, asthma, atopic dermatitis, autoimmune thyroid disorders, ulcerative colitis, Crohn's disease, Alzheimer's disease, leukemia and other indications, where immunosuppression would be desirable (see WO 03/048126), and is known under the INN tasocitinib. The 3-{(3R,4R)-4-methyl-3-[methyl-(7H-pyrrolo[2,3-d]pyrimidin-4-yl)-amino]-piperidin-1-yl}3-oxo-propionitrile apparently has the following chemical structure, formula (I):

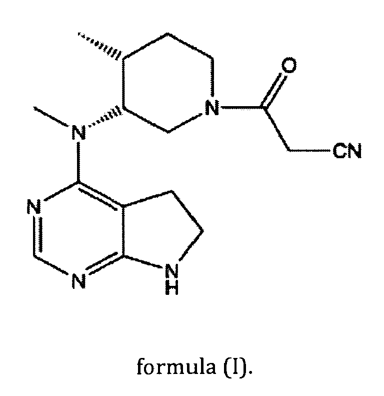

In this regard, it is reported that the compound according to formula (I), 3-{(3R,4R)-4-methyl-3-[methyl-(7H-pyrrolo [2,3-d] pyrimidin-4-yl)-amino]-piperidin-1-yl}3-oxo propionitrile, or its solvates or hydrates as well as pharmaceutical acceptable salts thereof are said to be obtained according to the procedures as outlined in WO 02/096909. The mono citrate form is said to be obtained in WO 03/048162.

Whereas the prior art (WO 03/048162, WO 02/096909) mentions that tasocitinib might be formulated into pharmaceutical compositions such as in the form of tablets, no specific immediate release formulations have been disclosed.

In addition, it has been reported that tasocitinib has some undesirable properties. For example, it turned out to be difficult to ensure blend and content uniformity in pharmaceutical dosage form.

Moreover, it turned out that it is difficult to press tasocitinib into tablets, since the resulting tablets have undesirable properties, e.g. tend to crumble and disintegrate. Further, it was observed that some process types could lead to an undesirable converting of polymorphous forms of the active ingredient tasocitinib. The wet granulation or wet compressing techniques are further detrimental for economic reasons, since they are energy- and time-consuming and cost-intensive.

It is therefore desired to develop a tasocitinib formulation that provides good processability, e.g. superior flowability and pourability. Further, the formulations should show superior dissolution properties and superior bioavailability. Still further, the formulations should have superior oxidation stability, as well as a superior storage stability and shelf life.

In addition, the dosage forms of the present invention should show a superior content uniformity. An inhomogeneous formulation can lead to overdosing or subdosing the active ingredient. In both cases the inhomogeneous formulation may lead to severe side effects and even intoxication. The requirement of uniformity of single-dose pharmaceutical formulations is described in the European Pharmacopoeia (hereinafter referred to as "Ph.Eur."), 6th edition, Chapter 2.9.6.

In conclusion, there is a need for the provision of pharmaceutical dosage forms and processes for the manufacture of these pharmaceutical dosage forms comprising tasocitinib, which do not suffer from the above mentioned draw-backs.

### Summary of the Invention

According to the present invention, the above objectives are achieved by specific tasocitinib formulations described herein. Furthermore, the above drawbacks can be overcome by a process for producing a pharmaceutical composition comprising the steps of
(i) providing
   a) tasocitinib,
   b) and at least one excipient,
(ii) blending components a) and b) to obtain a composition, wherein the D50 value of the particle size distribution is from 0.5 µm to 250 µm as measured by laser light diffraction.

A further subject of the present invention is a pharmaceutical composition, in particular a pharmaceutical dosage form, such as an oral pharmaceutical dosage form, preferably a tablet, obtainable by the process of the present invention.

In addition, a further subject of the present invention is a pharmaceutical composition, in particular a pharmaceutical dosage form such as an oral pharmaceutical dosage form, preferably a tablet, comprising
(a) 0.5 to 80 wt.% tasocitinib, based upon the total weight of the composition, and
(b) excipients, containing
from 10 to 95 wt.% of a filler,
from 1 to 30 wt.% of a disintegrant,
0 to 30 wt.% of a wicking agent,
0 to 30 wt.% solubilizer ,
0 to 10 wt.% of a lubricant, and
0 to 10 wt.% of a glidant, based upon the total weight of the composition.

The above illustrated subjects of the present invention are alternative solutions to the above outlined problems.

### Detailed Description of the Invention

In the following, explanations regarding the process of the present invention are given.

However, these explanations also apply to the pharmaceutical composition, in particular the pharmaceutical dosage form such as the oral pharmaceutical dosage form, preferably a tablet of the present invention and to the use of the present invention.

The term tasocitinib (a) as used in the present invention, refers to a compound according to formula (I), preferably in its acid form or its basic form. Tasocitinib (a) as used in the present invention also relates to the pharmaceutically acceptable acid addition salts as described in WO 02/096909. The acids which are used to prepare the pharmaceutically acceptable acid addition salts are preferably those which form non-toxic acid addition salts, i. e., salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [1,1'-methylene-bis-(2-hydroxy-3-naphthoate)] salts.

The invention also relates to stereospecific base addition salts of formula (I). The chemical bases that may be used as reagents to prepare pharmaceutically acceptable base salts of those compounds of formula I that are acidic in nature are those that form non-toxic base salts with such compounds. Such non-toxic base salts include, but are not limited to, those derived from such pharmacologically acceptable cations, such as alkali metal cations (e.g., potassium and sodium) and alkaline earth metal cations (e.g., calcium and magnesium), ammonium or water-soluble amine addition salts such as N-methylglucamine-(meglumine), and the lower alkanol ammonium and other base salts of pharmaceutically acceptable organic amines.

In the pharmaceutical composition of the present invention, tasocitinib as the active ingredient (component (a)) can be provided in amorphous form, in crystalline form or as a mixture of both forms. Preferably, tasocitinib is present in crystalline form, wherein the crystalline modification is as described in WO 03/048162. In a particularly preferred embodiment of the present invention, tasocitinib is provided as the citrate or hemi citrate. Most preferred is the crystalline form of the citrate or hemi citrate of tasocitinib.

In the present invention tasocitinib as the active ingredient (a) and the at least one excipient (b) are usually employed in a particulate form. Preferably, the D50 value of the particle size distribution of said particles is from 0.5 µm to 250 µm as measured by laser light diffraction. More preferably, the particles of the pharmaceutical composition of the present invention have a D50 value of the particle size distribution of 2.0 to 150 µm, still more preferably of 5.0 to 120 µm, especially more preferably of 10 to 90 µm and in particular from 15 to 70 µm.

Furthermore, in a preferred embodiment the D90 value of the particle size distribution is from 10 to 500 µm, preferably from 20 to 290 µm, more preferably from 30 to 180 µm.

Furthermore, in a preferred embodiment the D10 value of the volume mean particle size distribution is from 0.1 to 90 µm, preferably from 1.0 to 70 µm, more preferably from 2.0 to 50 µm.

Within this application, the particle size distribution is determined by the light scattering method, using a Mastersizer 2000 apparatus made by Malvern Instruments (wet measurement, 2000 rpm, ultrasonic waves for 60 sec., data interpretation via Fraunhofer Method, dispersant: liquid paraffin, stirrer speed: 2000 rpm, stirring duration: 15 min prior to first measurement cycle, sonication: no, background time: 10 sec, measurement time: 10 sec, measurement cycles: 3).

The D50 value of the integral volume distribution is defined in the context of this invention as the particle diameter, at which 50 percent by volume of the particles have a smaller diameter than the diameter which corresponds to the D50 value. Likewise, 50 percent by volume of the particles have a larger diameter than the D50 value. Analogously, the D90 value of the integral volume distribution is defined as the particle diameter, at which 90 percent by volume of the particles have a smaller diameter than the diameter, which corresponds to the D90 value. Correspondingly, the D10 value of the integral volume distribution is defined as the particle diameter, at which 10 percent by volume of the particles have a smaller diameter than the diameter, which corresponds to the D10 value.

The pharmaceutical composition comprises tasocitinib preferably in an amount of 0.5 to 80 wt.%, preferably between 1 and 20 wt.%, more preferably between 2 and 8 wt.%, based on the total weight of the composition.

The pharmaceutical composition further comprises at least one excipient (component (b) and/or (c)). Suitable excipients include, but are not limited to, fillers, solubilizers, lubricants, disintegrants, wicking agents, surfactants, glidants, anti-sticking agents and mixtures thereof.

Generally, fillers are used to top up the volume for an appropriate oral deliverable dose, when low concentrations of the active pharmaceutical ingredients (about 30 wt.% or lower) are present. However, in powder formulations fillers might be useful to enhance the powder flow, so as to guarantee, for example, a uniform filling of the capsules. Fillers are usually relatively chemically inert, but they can have an effect on the bioavailability of the active ingredient. They can influence the solubility of the active ingredient and enable a powder of an insoluble compound to break up more readily on capsule shell disintegration.

Preferred fillers of the invention are calcium phosphate, calcium carbonate, calcium silicate, magnesium carbonate, magnesium oxide, maltodextrin, glucopyranosyl mannitol, calcium sulfate, dextrate, dextrin, dextrose, saccharose, lactose, hydrogenated vegetable oil and/or cellulose derivatives, most preferably lactose. A pharmaceutical composition according to the invention may comprise an inorganic salt as a filler. Preferably, this inorganic salt is dicalcium phosphate, preferably in form of the dihydrate (dicafos). A preferred filler is 1-*0*-α-D-glucopyranosyl-D-mannitol (isomalt).

Usually, fillers can be used in an amount of 10 to 98 wt.%, preferably of 20 to 95 wt.%, more preferably 40 to 90 wt.%, in particular 60 to 85 wt.%, based on the total weight of the composition.

The composition of the subject invention preferably comprises one or more solubilizers, preferably hydrophilic solubilizers. Alternatively, the solubilizer could also be denoted as matrix former, i.e. the terms "solubilizer" and "matrix former" are used synonymously.

Generally, the term "solubilizer" means any organic excipient, which is capable of improving the solubility and/or dissolution of the active pharmaceutical ingredient. Generally, the term "hydrophilic solubilizer" means any organic excipient, which possesses hydrophilic groups and is capable of improving the solubility and/or dissolution of the active pharmaceutical ingredient. Preferably, the hydrophilic solubilizer is capable of reducing the dissolution time of a pharmaceutical composition by 5 %, more preferably by 20 %, according to USP 31-NF26 release method, using apparatus 2 (paddle), compared to the same pharmaceutical composition comprising calcium hydrogen phosphate instead of the hydrophilic solubilizer.

The solubilizers are selected, for example, from the group of known inorganic or organic excipients. Such excipients preferably include polymers, low molecular weight oligomers and natural products.

Preferably, the hydrophilic solubilizer is a water-soluble compound, having a water solubility of more than 10 mg/l, more preferably of more than 20 mg/l, still more preferably of more than 50 mg/l at a temperature of 25 °C. The solubility of the hydrophilic solubilizer might be e.g. up to 1,000 mg/l or up to 300 mg/ml at a temperature of 25 °C. The water-solubility is determined according to the column elution method of the Dangerous Substances Directive (67/548/EEC), Annex V, Chapter A6.

In a preferred embodiment the solubilizer is a hydrophilic polymer, preferably having the above mentioned water-solubility. Generally, the term "hydrophilic polymer" encompasses polymers comprising polar groups. Examples for polar groups are hydroxy, amino, amido, carboxy, carbonyl, ether, ester and sulfonate. Amido groups are particularly preferred.

The hydrophilic polymer can usually have a weight average molecular weight, ranging from 1,000 to 250,000 g/mol, preferably from 2,000 to 100,000 g/mol, particularly from 4,000 to 75,000 g/mol. Furthermore, a 2 % w/w solution of the hydrophilic polymer in pure water preferably has a viscosity of from 1 to 20 mPas, more preferably from 2 to 8 mPas at 25 °C. The viscosity is determined according to the European Pharmacopoeia, 6th edition, Chapter 2.2.10.

Furthermore, the hydrophilic polymer used as hydrophilic solubilizer preferably has a glass transition temperature (T_{g}) or a melting point of 25 °C to 200 °C, more preferably of 90 °C to 170 °C. The glass transition temperature, T_{g}, is the temperature at which the hydrophilic polymer becomes brittle on cooling and soft on heating. That means, above T_{g} the hydrophilic polymers become soft and capable of plastic deformation without fracture. The glass transition temperature or the melting point are determined with a Mettler-Toledo^{®} DSC 1, wherein a heating rate of 10 °C per minute and a cooling rate of 15 °C per minute is applied. The determination method essentially is based on Ph. Eur. 6.1, section 2.2.34. For the determination of T_{g} the polymer is heated twice (i.e. heated, cooled, heated).

More preferably, derivatives of cellulose (e.g., hydroxyproply methyl cellulose (HPMC), preferably having a weight average molecular weight from 20,000 to 90,000 g/mol, and/or preferably a ratio of methyl groups from 10 to 35 %, and preferably a ratio of hydroxypropyl groups from 1 to 35 %; hydroxypropyl cellulose (HPC), preferably having a weight average molecular weight of from 40,000 to 100,000 g/mol), polyvinylpyrrolidone, preferably having a weight average molecular weight of from 10,000 to 60,000 g/mol, copolymers of polyvinylpyrrolidones, preferably copolymers comprising vinylpyrrolidone and vinylacetate units (e.g. Povidon^{®} VA 64; BASF), preferably having a weight average molecular weight of 40,000 to 75,000 g/mol, polyoxyethylene alkyl ethers, co-blockpolymers of ethylene oxide and propylene oxide, preferably having a polyethylene content of 70 to 90 wt.% and/or preferably having a weight average molecular weight from 1,000 to 50,000 g/mol, in particular from 3,000 to 25,000 g/mol, polyvinyl alcohol, polyethylene glycol, preferably having a weight average molecular weight ranging from 1,000 to 50,000 g/mol, are used as hydrophilic solubilizers. The weight average molecular weight is preferably determined by gel electrophoresis.

In particular, polyvinylpyrrolidone and copolymers of polyvinylpyrrolidone, in particular copolymers comprising vinylpyrrolidone and vinylacetate units having the structure 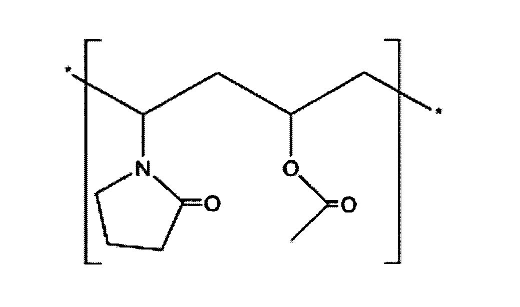 are used as hydrophilic solubilizers.

It is particularly preferred that the above mentioned kinds of hydrophilic polymers fulfill the functional requirements (molecular weight, viscosity, T_{g}, melting point, non-semipermeable properties) as illustrated above.

In the pharmaceutical composition of the present invention, at least one of the above mentioned hydrophilic solubilizers is present. Alternatively, a combination of two or more hydrophilic solubilizers can be employed.

Usually, solubilizers can be used in an amount of 0.1 to 30 wt.%, preferably of 1 to 20 wt.%, more preferably 2 to 12 wt.%, based on the total weight of the composition.

The composition of the present invention may further comprise one or more disintegrants. In a further embodiment of the present invention, the complete amount of the disintegrant(s) is added in two portions, namely a first disintegrant portion as well as a second disintegrant portion. Consequently, the oral dosage form, preferably the tablet of the present invention, also comprises a first disintegrant portion as well as a second disintegrant portion. The first and second disintegrant portion can comprise the same or different disintegrants.

Generally, disintegrants are compounds, capable of promoting the break up of a solid composition into smaller pieces when the composition gets in contact with a liquid, preferably water.

Preferred disintegrants are sodium carboxymethyl starch, cross-linked polyvinylpyrrolidone (crospovidone), sodium carboxymethyl glycolate (e.g. Explotab^{®}), swelling polysaccharide, e.g. soya polysaccharide, carrageenan, agar, pectin, starch and derivates thereof, protein, e.g. formaldehyde - casein, sodium bicarbonate or mixtures thereof. Crospovidone is particularly preferred as disintegrant. Furthermore, a combination of crospovidone and agar is particularly preferred.

Usually, disintegrants can be used in an amount of 0.1 to 20 wt.%, preferably of 1 to 10 wt.%, based on the total weight of the composition.

The pharmaceutical composition of the present invention may further comprise one or more wicking agent(s). The complete amount of wicking agent can be added in one portion or in two separate portions, namely a first wicking agent portion as well as a second wicking agent portion. Consequently, the oral dosage form, preferably the tablet of the present invention, also comprises a first wicking agent portion as well as a second wicking agent portion. The first and second wicking agent portion can comprise the same or different wicking agents.

Generally, a wicking agent is a material with the ability to draw a biological fluid (preferably water) into a solid (preferably into the agglomerates resulting from step (iii) of the process of the present invention), preferably by physisorption. Physisorption is defined as a form of adsorption, in which the solvent molecules can loosely adhere to the surfaces of the wicking agent, preferably via van der Waals interaction between the surface of the wicking agent and the adsorbed fluid molecule (preferably water). Usually, a wicking agent can do this with or without swelling. Preferably, the wicking agent is a swelling wicking agent. Usually, a non-swelling wicking agent that attracts water will ultimately have a volume that is essentially composed of the volume of the wicking agent and the volume of water attracted to it. Usually, a swelling wicking agent will have a volume that is essentially composed of the volume of the wicking agent, the volume of water attracted to it, and an additional volume created by steric and molecular forces. Preferably, the wicking agent comprised in the pharmaceutical composition of the present invention creates channels or pores in the agglomerates. This facilitates the channeling of water molecules through the agglomerates, particularly by physisorption. Hence, the function of the wicking agent is to carry water to surfaces inside the agglomerates, thereby creating channels or a network of increased surface area.

Examples of wicking agents that may be used include, but are not limited to, microcrystalline cellulose, silicified microcrystalline cellulose, colloidal silicone dioxide, kaolin, titanium dioxide, fumed silicone dioxide, alumina, niacinamide, m-pyrol, bentonite, magnesium aluminum silicate, polyester, polyethylene or mixtures thereof. Preferably, the wicking agents used in the pharmaceutical composition of the present invention include cellulose and cellulose derivatives, such as microcrystalline cellulose, silicified microcrystalline cellulose, colloidal silicone dioxide, and mixtures thereof. The silicified microcrystalline cellulose that is preferred is commercially available under the trade name Prosolv^{®}, having a silicone dioxide content from 1 to 3 wt.%, preferably of about 2 wt.%.

Usually, wicking agents can be used in an amount of 0.1 to 20 wt.%, preferably of 1 to 10 wt.%, based on the total weight of the composition.

The pharmaceutical composition of the present invention may further comprise one or more of a surfactant. Preferably, sodium lauryl sulfate is used as surfactant.

Usually, surfactants can be used in an amount of 0.05 to 2 wt.%, preferably of 0.1 to 1.5 wt.%, based on the total weight of the composition.

Additionally, the pharmaceutical composition, preferably the pharmaceutical dosage form, may comprise one or more additional excipients as for example: a lubricant, a glidant and/or an anti-sticking agent.

In a preferred embodiment of this invention, a lubricant may be used. Lubricants are generally employed to reduce dynamic friction. The lubricant preferably is a stearate, talcum powder or fatty acid, more preferably, hexanedioic acid or an earth alkali metal stearate, such as magnesium stearate. The lubricant is suitably present in an amount of 0.1 to 5 wt.%, preferably about 1.0 to 2.5 wt.% of the total weight of the composition. Preferably, the lubricant is applied in a final lubrication step during the powder preparation. The lubricant generally increases the powder flowability.

The glidant can for example be colloidal silicone dioxide (e.g. Aerosil^{®}). Preferably, the glidant agent is present in an amount of 0 to 8 wt.%, more preferably at 0.1 to 3 wt.% of the total weight of the composition. Preferably, the silicone dioxide has a specific surface area of 50 to 400 m²/g, measured according to Ph. Eur., 6th edition, Chapter 2.9.26, multipoint method, volumetric determination.

The anti-sticking agent is for example talcum and may be present in amounts of 0.05 to 5 wt.%, more preferably in an amount of 0.5 to 3 wt.% of the total weight of the composition.

In this context, it is generally noted that, due to the nature of pharmaceutical excipients, it cannot be excluded that a certain compound meets the functional requirements of more than one of the above mentioned excipient classes. However, in order to enable an unambiguous distinction and terminology in the present application, the same pharmaceutical compound can only be subsumed as one of the functional excipient classes presented above. For example, if dicalcium phosphate dihydrate is described as a filler, it cannot additionally classify as a solubilizer or as a disintegrant.

In a preferred embodiment of the invention, the composition comprises tasocitinib, one or more of a filler, one or more of a disintegrant, one or more of a wicking agent and one or more of a lubricant.

In a further preferred embodiment, the composition comprises from 60 to 95 wt.% of a filler, from 5 to 30 wt.% of a disintegrant, from 1 to 10 wt.% of a wicking agent and from 1 to 10 wt.% of a lubricant, based upon the total weight of the composition.

Generally, it is noted that all comments made above regarding the excipients of the present invention also apply not only for the process but also for the dosage form, preferably tablet of the present invention.

The process for producing the pharmaceutical composition of the present invention comprises the steps of
(i) providing
   a) tasocitinib,
   b) and at least one excipient,
(ii) blending components a) and b) to obtain a composition, wherein the D50 value of the particle size distribution of the blend is from 0.5 µm to 250 µm as measured by laser light diffraction,
(iii) optionally agglomerating the pharmaceutical composition into granules, and
(iv) further optionally compressing the composition from step (ii) or the granules from step (iii) into tablets or filling the composition from step (ii) or the granules from step (iii) into dosage forms like sachets or capsules.

The subject invention further provides oral pharmaceutical dosage forms obtainable by the processes as described herein. Such oral pharmaceutical dosage forms include, but are not limited to, tablets, sachets and capsules. In the preferred embodiment, the oral pharmaceutical dosage form is a tablet. Thus, in a preferred embodiment of the present invention the composition comprising tasocitinib is compressed into tablets.

Moreover, in the process of the invention one or more additional excipients may be added to the blended composition of step (ii), to the agglomeration step (iii) and/or to step (iv). Preferably, additional excipients are added in step (ii) and (iii), in particular in step (ii). The one or more excipients preferably have a surface area of 0.3 to 10 m²/g, preferably between 1 to 5 m²/g, as measured by gas adsorption according to Ph. Eur., 6th edition, Chapter 2.9.26, multipoint method, volumetric determination.

In a preferred embodiment, components a) and/or b) are micronized in order to obtain particles having the particle size as required by the present invention. Micronization can be carried out by milling such as in a jet air mill.

Blending (ii) of tasocitinib and the at least one excipient can be carried out with conventional mixing devices, e.g. in a free fall mixer like Turbula^{®} T 10B (Bachofen AG, Switzerland). Blending can be carried out e.g. for 1 minute to 30 minutes, preferably for 2 minutes to less than 10 minutes.

Generally, the term "agglomeration" refers to a process, wherein particles are attached to each other, thereby giving larger particles. The attachments may occur through physical forces, preferably van der Waals forces. The attachment of particles preferably does not occur through chemical reactions.

Agglomeration (iii) can be carried out in different devices. For example, agglomeration can be carried out by a granulation device, preferably by a dry granulation device. More preferably, agglomeration can be carried out by intensive blending. For example, agglomeration can be carried out by blending in a free-fall mixer or a container mixer. An example for a suitable free fall mixer is Turbula^{®} T10B (Bachofen AG, Switzerland). Generally, the blending is carried out for a time being long enough for agglomeration to occur. Usually, blending is carried out for 10 minutes to 2 hours, preferably for 15 minutes to 60 minutes, more preferably from 20 minutes to 45 minutes.

In a further preferred embodiment agglomeration is carried out by granulation, in particular by slugging or by roller compaction. Roller compaction is particularly preferred. When roller compaction is used, the compaction force usually ranges from 1 to 30 kN/cm, preferably from 2 to 20 kN/cm, more preferably from 3 to 10 kN/cm.

In a preferred embodiment the agglomeration step is carried out as a dry-agglomeration step. That means, the agglomeration step is carried out in the absence of solvents, preferably in the absence of organic solvents and/or in the absence of water.

In a preferred embodiment the agglomeration conditions in step (iii) are chosen such that the resulting agglomerated pharmaceutical composition comprises a volume mean particle size (D₅₀) of 5 to 500 µm, more preferably of 20 to 250 µm, further more preferably of 50 to 200 µm.

The bulk density of the agglomerated pharmaceutical composition made by the process of the present invention generally ranges from of 0.1 to 0.85 g/ml, preferably of from 0.25 to 0.85 g/ml, more preferably of from 0.3 to 0.75 g/ml.

The Hausner factor of the agglomerated composition is less than 1.3, preferably less than 1.2 and most preferably less than 1.15. The agglomerated pharmaceutical composition resulting from step (iii) of the invention preferably possesses Hausner ratios in the range of 1.02 to 1.5, preferably of 1.05 to 1.4, more preferably between 1.08 to 1.3. The Hausner ratio is the ratio of tapped density to bulk density. Bulk density and tapped density are determined according to USP 24, Test 616 "Bulk Density and Tapped Density".

As already mentioned above, the process for producing tablets according to the present invention may be characterized by splitting the amount of disintegrant into two portions (1) and (2). In a preferred embodiment the weight ratio of portion (1) : portion (2) is from 15 : 85 to 70 : 30, more preferably from 25 : 75 to 60 : 40.

In addition, the process for producing tablets according to the present invention preferably can be characterized by splitting the amount of the wicking agent into two portions (1) and (2). In a preferred embodiment the weight ratio of component (1): component (2) is from 10 : 60 to 60 : 40, more preferably from 20 : 50 to 55 : 45.

In addition, after the steps (ii), (iii) and/or (iv) preferably one or more further excipient(s), such as fillers, lubricants, glidants and anti-sticking agents, can be added. Regarding the above mentioned pharmaceutically acceptable excipients, the application generally refers to "Lexikon der Hilfsstoffe für Pharmazie, Kosmetik und angrenzende Gebiete", edited by H. P. Fiedler, 5th Edition, Editio Cantor Verlag, Aulendorf and earlier editions, and "Handbook of Pharmaceutical Excipients", third edition, edited by Arthur H. Kibbe, American Pharmaceutical Association, Washington, USA, and Pharmaceutical Press, London.

In a preferred embodiment the composition resulting from step (ii) or the granules resulting from step (iii) is compressed into tablets or filled into dosage forms like sachets or capsules (step (iv)). Preferably, the composition is pressed into tablets. Compression of tablets can be achieved by direct compressing the pharmaceutical composition of the invention, by dry compaction, wet compaction or melt granulation. The compression step (iv), preferably a direct compression step, is preferably carried out on a rotary press, e.g. on a Fette^{®} 102i (Fette GmbH, Germany) or a Riva^{®} piccola (Riva, Argentina).

If a rotary press is applied, the main compression force usually ranges from 1 to 50 kN, preferably from 2 to 40 kN, more preferably from 3.5 to 30 kN. The resulting tablets usually have a hardness as described below.

Consequently, further subjects of the present invention are tablets obtainable by any of the processes as described above. Hence, a further subject of the present invention is a pharmaceutical composition comprising
(a) 0.5 to 80 wt.% tasocitinib, based upon the total weight of the composition, and
(b) excpients, containing
from 10 to 95 wt.% of a filler,
from 1 to 30 wt.% of a disintegrant,
0 to 30 wt.% of a wicking agent,
0 to 30 wt.% solubilizer,
0 to 10 wt.% of a lubricant, and
0 to 10 wt.% of a glidant, based upon the total weight of the composition.

All explanations regarding excipients given above for the process of the present invention also apply for the dosage form of the present invention.

In addition, subject of the present invention is a tablet comprising
(I) an inner phase, such as an intragranular phase, containing
   a) tasocitinib,
   b) at least one excipient, and
(II) an outer phase, such as an outergranular phase, containing
   c) at least one excipient.

In a preferred embodiment, subject of the present invention is a tablet comprising
(I) an inner phase containing
   a) tasocitinib,
   b1) a filler,
   b2) optionally a disintegrant,
   b3) optionally a wicking agent,
   b4) optionally a solubilizer,
   b5) optionally a glidant, and
   b6) optionally further excipients, and
(II) an outer phase containing
   c1) a filler,
   c2) a disintegrant,
   c3) optionally a wicking agent,
   c4) optionally a solubilizer,
   c5) optionally a glidant, and
   c6) optionally further excipients.

Especially, the tablets can comprise
(I) an inner phase containing
   a) 0.5 to 80 wt.% tasocitinib,
   b1) 5 to 90 wt.% filler,
   b2) 0 to 30 wt.% disintegrant,
   b3) 0 to 30 wt.% wicking agent,
   b4) 0 to 30 wt.% a solubilizer,
   b5) 0 to 10 wt.% glidant, and
   b6) optionally further excipients, and
(II) an outer phase containing
   c1) 5 to 90 wt.% filler,
   c2) 0 to 30 wt.% disintegrant,
   c3) 0 to 30 wt.% wicking agent,
   c4) 0 to 30 wt.% solubilizer,
   c5) 0 to 10 wt.% glidant, and
   c6) optionally further excipients, wherein all weight percent are based on the total weight of the tablet. If the tablet is coated, all weight percents are based on the total weight of the uncoated tablet.

Thus, it is preferred that the tablet of the invention comprises an inner phase, such as an intragranular phase, comprising tasocitinib, and an outer phase, such as an extragranular phase, wherein the inner phase preferably further comprises a filler, a disintegrant and a glidant, and the outer phase preferably comprises a filler, a wicking agent and a glidant.

All explanations above given for the process of the present invention also apply for the tablet of the present invention. That means, the inner phase of the tablet of the present invention preferably is produced as described above for steps (i) and (ii) and optionally (iii), the outer phase preferably is produced by blending the compounds (c1) and optionally one or more of components (c2), (c3), (c4), (c5) and (c6) with the inner phase, as described in step (ii) and optionally (iii) and subsequently compressing that mixture, as described in step (iv).

The tablets of the present invention can be film-coated tablets for peroral use or dispersing tablets. Film-coated tablets for peroral use are preferred.

The film-coating agent is for example hydroxypropyl methyl cellulose or methacrylate and may be present in an amount of 1 to 10 wt.%, more preferably in an amount of 2 to 8 wt.%, based on the total weight of the composition. The thickness of the film usually ranges from 1 to 80 µm, preferably from 4 to 60 µm.

The pharmaceutical compositions and oral dosage forms (e.g. tablets) of the present invention are formulations showing "immediate release". Within the scope of this patent application, immediate release formulations have a Q value of not less than 75 %, preferably have a Q value of from 80 % to 100 %, more preferably a Q value of from 90 % to 100 %, in particular, a Q value from 92 to 100 %. The Q value is determined as described in USP 32-NF 27 method II (paddle, chapter <711>). In case of tablets these values refer to the uncoated tablet.

Furthermore, the pharmaceutical compositions and tablets of the present invention preferably do not comprise compounds imparting modified release properties. More preferably, the pharmaceutical compositions and tablets of the present invention do not comprise a modified release system comprising a non-erodible polymer.

Preferably, the tablets of the present invention usually have a hardness of from 50 to 300 Newton (N), more preferred from 60 t 250 N, still more preferred from 70 to 220 N, in particular, from 80 to 180 N, wherein the hardness is measured according to Ph.Eur. 6.0, Chapter 2.9.8.

Further, the tablets of the present invention preferably have a friability of less than 3 %, more preferably of less than 2 %, still more preferably less than 1 %, in particular, 0.9 to 0.1 %, wherein the friability is measured according to Ph.Eur. 6.0, Chapter 2.9.7.

Further, the tablets of the present invention usually have a *Content Uniformity* from 95 to 105 %, preferably from 98 to 102 %, in particular from 99 to 101 % (That means, all tablets have a drug content from 95 to 105 %, preferably from 98 to 102 %, in particular fro 99 to 101 %, wherein the average drug content of all tablets is set as 100 %). The *Content Uniformity* is determined according to Ph. Eur.6.0, Chapter 2.9.6.

The present invention also provides the use of the pharmaceutical composition or the oral dosage form of the present invention as an immunosuppressive agent for organ transplants, xenotransplantation, lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia. The pharmaceutical composition or the oral dosage form of the present invention can be used as an immunosuppressive agent in a method for organ transplants or xenotransplantation, or for treating lupus, multiple sclerosis, rheumatoid arthritis, psoriasis, Type I diabetes and complications from diabetes, cancer, asthma, atopic dermatitis, autoimmune thyroid disorders, ulcerative colitis, Crohn's disease, Alzheimer's disease, leukemia, said method comprising administering an effective amount of the pharmaceutical composition or the oral dosage form in a subject in need thereof.

The present invention is illustrated by the following examples.

### EXAMPLES

The following commercially available compounds were used in the examples below:
- MicroceLac^{®} 100 (Meggle):: spray dried composition of 75 wt.% lactose monohydrate and 25 wt.% microcrystalline cellulose
- Kollidon^{®} CL (BASF):: crosslinked polyvinyl pyrrolidone (Crospovidone)
- Aerosil^{®} (Degussa):: highly dispersed silicium dioxide
- GalenIQ^{®} (BENEO-Palatinit):: 1-0-α-D-Glucopyranosyl-D-mannitol (isomalt)
- Avicel^{®} 101 (FMC):: microcrystalline cellulose

### Example 1: direct compression (a)

**Tablet formulation 1:**

| | |
|---|---|
| Tasocitinib citrate | 5 mg (based on the free base) |
| MicroceLac^{®} 100 | 81 mg |
| Kollidon^{®} CL | 10 mg |
| Aerosil^{®} | 2 mg |
| Magnesium stearate | 2 mg |

Tasocitinib and MicroceLac^{®} 100 were sieved (800 µm mesh) and pre-mixed in a Turbula^{®} mixer for 15 min. Then Kollidon^{®} CL and Aerosil^{®} were sieved (800 µm mesh) and added to the pre-mixture and then further blended in a Turbula^{®} mixer for 10 min. Magnesium stearate was added and the resulting mixture was blended in a free fall mixer for 3 min. The final blend was compressed into tablets with an excenter press (Korsch EK0), using a 6 mm rotary biconvex punch. The tablets had a hardness of 50 to 85 N.

**Tablet formulation 2:**

| | |
|---|---|
| Tasocitinib hemi citrate | 5 mg (based on the free base) |
| GalenIQ^{®} | 81 mg |
| Kollidon^{®} CL | 10 mg |
| Aerosil^{®} | 2 mg |
| Magnesium stearate | 2 mg |

The tablets were prepared as described for tablet formulation 1, except that GalenIQ^{®} was used instead of MicroceLac^{®} 100.

### Example 2: Dry compaction (b)

**Tablet formulation 3:**

| | |
|---|---|
| Tasocitinib hemi citrate | 5 mg (based on the free base) |
| GalenIQ^{®} | 71 mg |
| Kollidon^{®} CL | 10 mg |
| Kollidon^{®} CL-M | 10 mg |
| Aerosil^{®} | 2 mg |
| Magnesium stearate | 2 mg |

Tasocitinib, and 2/3 of GalenIQ^{®} and Kollidon^{®} CL-M were compacted and sieved (800 µm mesh) to produce an inner phase. Then the residual GalenIQ^{®}, Kollidon^{®} CL-M and Aerosil^{®} were sieved (800 µm mesh) and added to produce an outer phase. The blend was mixed for 15 min in a Turbula^{®} mixer for 15 min. Magnesium stearate was added and the resulting mixture was blended in a free fall mixer for 3 min. The final blend was compressed into tablets with an excenter press (Korsch EK0), using a 6 mm rotary biconvex punch. The tablets had a hardness of 50 to 85 N.

**Tablet formulation 4:**

| | |
|---|---|
| Tasocitinib citrate | 5 mg (based on the free base) |
| Avicel^{®} 101 | 35 mg |
| Lactose monohydrate | 36 mg |
| Kollidon^{®} CL | 10 mg |
| Kollidon^{®} CL-M | 10 mg |
| Aerosil^{®} | 2 mg |
| Magnesium stearate | 2 mg |

Tasocitinib, Avicel® 101, lactose monohydrate and Kollidon^{®} CL were pre-mixed, compacted and sieved (800 µm mesh). Then Kollidon^{®} CL and Aerosil^{®} were sieved (800 µm mesh), added to the pre-mixture and then further blended in a Turbula^{®} mixer for 15 min. Magnesium stearate was added and the resulting mixture was blended in a free fall mixer for 3 min. The final blend was compressed into tablets with an excenter press (Korsch EK0), using a 6 mm rotary biconvex punch. The tablets had a hardness of 50 to 85 N.

The methods of direct compression and dry compaction are further beneficial since they can go without heating or melting the pharmaceutical composition, which could lead to decomposing the active ingredient. Accordingly, these methods have the advantage that tasocitinib is subjected to lower thermal strain compared to wet granulation or melt granulation.

## Claims

1. Process for producing a pharmaceutical composition, comprising the steps of
(i) providing
a) tasocitinib, and
b) at least one excipient,
(ii) blending components a) and b) to obtain a particulate composition, wherein the D50 value of the particle size distribution is from 0.5 µm to 250 µm as measured by laser light diffraction.

2. Process according to claim 1, further comprising a step (iii) of agglomerating the pharmaceutical composition into granules.

3. Process according to claim 1 or 2, further comprising a step (iv), wherein the composition from step (ii) or the granules from step (iii) are compressed into tablets or filled into sachets or capsules, preferably compressed into tablets.

4. Process according to any one of the previous claims, wherein one or more additional excipients (c) are added to step (ii), (iii) or (iv).

5. Process according to any one of claims 2 to 4, wherein the agglomeration step is carried out in a granulator or mixer, preferably in a free-fall mixer.

6. Process according to any one of the previous claims, wherein the excipients comprise fillers, solubilizers, lubricants, disintegrants, wicking agents, surfactants, glidants, anti-sticking agents and mixtures thereof.

7. Process according to any one of the previous claims, wherein the one or more excipients have a surface area of 0.3 to 10 m²/g, preferably between 1 to 5 m²/g, as measured by gas adsorption according to Ph. Eur., 6th edition, Chapter 2.9.26, multipoint method, volumetric determination.

8. Pharmaceutical composition, obtainable by a process according to any one of the preceding claims, which preferably is a tablet, sachet or capsule, most preferably a tablet.

9. Pharmaceutical composition comprising
(a) 0.5 to 80 wt.% tasocitinib, based upon the total weight of the composition, and
(b) excpients, containing
from 10 to 95 wt.% of a filler,
from 1 to 30 wt.% of a disintegrant,
0 to 30 wt.% of a wicking agent,
0 to 30 wt.% solubilizer,
0 to 10 wt.% glidant, and
0 to 10 wt.% of a lubricant, based upon the total weight of the composition.

10. Pharmaceutical composition according to claim 9 in form of a tablet, comprising
(I) an inner phase, containing
a) tasocitinib,
b) at least one excipient, and
(II) an outer phase, containing
c) at least one excipient.

11. Pharmaceutical composition according to claim 9 or 10, comprising
(I) an inner phase, containing
a) tasocitinib,
b1) a filler,
b2) a disintegrant,
b3) optionally a wicking agent,
b4) optionally a solubilizer,
b5) optionally a glidant, and
(II) an outer phase containing
c1) a filler,
c2) a disintegrant,
c3) optionally a wicking agent,
c4) optionally a solubilizer and
c5) optionally a glidant.

12. Pharmaceutical composition according to any one of claims, 9 to 11, wherein tasocitinib is present in amorphous form or in crystalline form, preferably in crystalline form, most preferably as crystalline tasocitinib citrate or hemi citrate.

13. Pharmaceutical composition according to any one of claims 9 to 12 in form of an immediate release tablet.

14. Tablet according to claim 13, having a hardness of from 50 to 300 Newton (N), a friability of less than 3 % and a content uniformity of 95 to 105 % .
